# EUROPEAN PATENT SPECIFICATION

(11) **EP 2 348 007 B1**
(45) Date of publication and mention of the grant of the patent: **17.10.2018**
(21) Application number: 11156860.6
(22) Date of filing: 03.11.2006
(51) Int. Cl.: C07C 17/23, C07C 17/25, C07C 17/278, C07C 21/18, C07C 19/10

(54) **PROCESS FOR THE PREPARATION OF CF3CF2CH2CL**
VERFAHREN ZUR HERSTELLUNG VON CF3CF2CH2CL
PROCÉDÉ POUR LA PRÉPARATION DU CF3CF2CH2CL

(30) Priority: 03.11.2005 US 733378 P
(43) Date of publication of application: 27.07.2011
(62) Divisional of application: 06827435.6
(73) Proprietor: Honeywell International Inc., Morris Plains, NJ 07950 (US)
(72) Inventor: Mukhopadhyay, Sudip, Williamsville, NY 14221 (US); Dubey, Rajesh, Buffalo, NY 14210 (US); Ratna Singh, Rajiv, Getzville, NY 14068 (US); Shia, George, Morristown, NJ 07962-2245 (US)
(74) Representative: Crooks, Elizabeth Caroline

(56) References cited:
- WO-A-90/08748
- WO-A-98/42645
- WO-A-2005/042451
- WO-A1-2006/124335
- GB-A- 1 171 202
- US-A1- 3 505 417
- US-A1- 2006 217 577
- HASZELDINE, R. N. ET AL: "Addition of free radicals to unsaturated systems. XIII. Direction of radical addition to chloro-1,1-difluoroethylene", JOURNAL OF THE CHEMICAL SOCIETY, 1957, pages 2193-2197, XP009081235,
- V. MONTANARI: "A Novel Synthesis of Perhalogenated Alkenes", J. ORG. CHEM., vol. 57, 1992, pages 5018-5019, XP002426455,
- R. S. DICKSON; G. D. SUTCLIFFE: "Fluorocarbon-Aluminium Compounds", AUST. J. CHEM., vol. 25, 1972, pages 761-768, XP009081192,
- MCDONIEL, J. BRIDGET ET AL: "Threshold Energy and Unimolecular Rate Constant for Elimination of HF from Chemically Activated CF3CF2CH3: Effect of the CF3 Substituent on the .alpha.-Carbon", JOURNAL OF PHYSICAL CHEMISTRY A, vol. 101, no. 7, 1997, pages 1334-1337, XP002426456,
- HASZELDINE, R.N. ET AL: "Free-radical additions to unsaturated systems. XVII. Reaction of trifluoroiodomethane with mixtures of ethylene and vinyl fluoride and of ethylene and propene", JOURNAL OF THE CHEMICAL SOCIETY, SECTION C: ORGANIC CHEMISTRY, CHEMICAL SOCIETY. LETCHWORTH, GB, vol. 3, 1970, pages 414-421, XP002343900, ISSN: 0022-4952, DOI: DOI:10.1039/J39700000414
- BELEN'KII G G ET AL: "Electrophilic, catalytic alkylation of polyfluoroolefins by some fluoroalkanes", JOURNAL OF FLUORINE CHEMISTRY, ELSEVIER, NL, vol. 108, no. 1, 2001, pages 15-20, XP004231215, ISSN: 0022-1139, DOI: DOI:10.1016/S0022-1139(00)00408-5
- PALETA, O. ET AL.: "Synthesis of perfluoroallyl chloride and some chlorofluoropropenes", BULLETIN DE LA SOCIETE CHIMIQUE DE FRANCE, SOCIETE FRANCAISE DE CHIMIE. PARIS, FRANCE, no. 6, 1986, pages 920-924, XP009088473, ISSN: 0037-8968
- TANUMA, T. ET AL.: "19F nuclear magnetic resonance studies of halogenated propanes", JOURNAL OF FLUORINE CHEMISTRY, ELSEVIER, NL, vol. 57, 1992, pages 259-284, XP002546058, ISSN: 0022-1139
- PALETA, O. ET AL.: "ADDITION REACTIONS OF HALOOLEGINS. XI. REACTION OF TETRAFLUOROETHYLENE WITH MONOFLUOROMETHANES IN THE PRESENCE OF ALUMINUM CHLORIDE", COLLECTION OF CZECHOSLOVAK CHEMICAL COMMUNICATIONS, vol. 36, 1971, pages 1867-1875, XP009071825, ISSN: 0010-0765
- POSTA A ET AL: "Reaction of Trifluoroethylene with Fluorochloromethanes in the Presence of Aluminium Chloride", COLLECTION OF CZECHOSLOVAK CHEMICAL COMMUNICATIONS, vol. 39, 1974, pages 1330-1335, XP009148472, ISSN: 0010-0765
- PALETA O: "Addition Reactions of Haloolefins. X. The Reactivity of Monofluorochloromethanes and Influence of Solvents on the Addition Rate of Halogenomethanes", COLLECTION OF CZECHOSLOVAK CHEMICAL COMMUNICATIONS, vol. 36, 1971, pages 2062-2066, XP009148480, ISSN: 0010-0765

## Description

### BACKGROUND OF INVENTION

### (1) Field of Invention:

This invention relates to novel methods for preparing CF₃CF₂CH₂Cl.

### (2) Description of Related Art:

Hydrofluorocarbons (HFC's), in particular hydrofluoroalkenes such tetrafluoropropenes (including 2,3,3,3-tetrafluoro-1-propene (HFO-1234yf) and 1,3,3,3-tetrafluoro-1-propene (HFO-1234ze)) (HFO is hydrofluorolefin) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkanes are known. For example, U.S. Pat. No. 4,900,874 (Ihara et al) describes a method of making fluorine containing olefins by contacting hydrogen gas with fluorinated alcohols. Although this appears to be a relatively high-yield process, for commercial scale production the handling of hydrogen gas at high temperature raises difficult safety related questions. Also, the cost of producing hydrogen gas, such as building an on-site hydrogen plant, can be in many situations prohibitive.

U.S. Pat. No. 2,931,840 (Marquis) describes a method of making fluorine containing olefins by pyrolysis of methyl chloride and tetrafluoroethylene or chlorodifluoromethane. This process is a relatively low yield process and a very large percentage of the organic starting material is converted in this process to unwanted and/or unimportant byproducts, including a sizeable amount of carbon black. The carbon black is not only unwanted, it tends to deactivate the catalyst used in the process.

The preparation of HFO-1234yf from trifluoroacetylacetone and sulfur tetrafluoride has been described. *See* Banks, et al., Journal of Fluorine Chemistry, Vol. 82, Iss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.
Catalyzed hydrogen reduction reactions have been disclosed for the preparation of fluorinated C3 hydrocarbons in U.S. Patent No. 5,545,777. The patent describes the reaction as being one in which a compound of formula (1)

C₃HₐCl_{b}F_{c} (1)

is converted by catalyzed hydrogen reduction to a compound of formula (2)

C₃Hₐ₊ₓCl_{b-y}F'_{c-z} (2)

where a, b, c, x, y and z are integers satisfying the following conditions: a≥0, b≥1, c≥2, x≥1, y≥1, z≥0, a+b+c=8, x=y+z, b-y≥0, and c-z≥2. Since the reactions disclosed in this patent require a reaction product in which a+b+c=8 and that x=y+z, it is not possible for the disclosed reaction product to include C3 olefins, which as mentioned above have been found to be desirable for use in many important applications.
Haszeldine et al (J. Chem. Soc., 1957, 2193-2197) report radical addition of compounds to 2-chloro-1,1-difluoroethylene. GB-1,171,202 discloses the preparation of CF₃CF₂CH₂Cl from CF₃CF₂CH₃ and Cl₂. Paleta O. reported the reaction of TFE with monofluoromethanes and, separately, the reactivity of monofluorochloromethanes (Czechoslov. Chem. Comm., vol 36, 1971, pages 1867-1875 and 2062-2066).

Notwithstanding prior teachings applicants appreciate a continuing need for methods of efficiently preparing CF₃CF₂CH₂Cl, which can be used to prepare HFO-1234yf.

### SUMMARY OF THE INVENTION

Applicants have discovered a method for producing CF₃CF₂CH₂Cl.

CF₃CF₂CH₂Cl can be converted into 1234yf. The preferred converting step of the present invention comprises catalytic reduction of CF₃CF₂CH₂Cl. The catalytic reduction step comprises in preferred embodiments introducing said compound CF₃CF₂CH₂Cl to a reaction system under conditions effective to convert, and preferably convert at least 50%, more preferably at least 70%, and even more preferably at least 90%, of said compound CF₃CF₂CH₂Cl. It is also generally preferred that said converting step produces a reaction product having at least 20% selectivity, more preferably at least 40% selectivity and even more preferably at least 70% selectivity, to HFO-1234yf.

In certain preferred embodiments, the converting step comprises reacting CF₃CF₂CH₂Cl in the gas phase, in the liquid phase, or a combination of these, with gas phase reactions preferably occurring in the presence of catalyst.

### DETAILED DESCRIPTION OF PREFERRED EMBODIMENTS

One beneficial aspect of the present invention is that it enables the production of desirable fluroolefins, such as 1234yf, using relatively high conversion and high selectivity reactions. In addition, the methods of the present invention provided reactions with relatively high yield and which are capable of obtaining relatively long catalyst life.

Furthermore, the present methods in certain preferred embodiments permit the production of desirable fluoroolefins from relatively attractive starting materials. Tetrafluoroethylene is an advantageous starting material because it is relatively easy to handle, and is generally readily available in commercial quantities and/or can be easily produced from other readily available materials. For example, CF₃CF₂CH₂Cl can be synthesized by the catalyzed gas phase addition of CH₂FCl and CF₂=CF₂.

The reaction by which TFE is converted to CF₃CF₂CH₂Cl is sometimes referred to herein for convenience, but not necessarily by way of limitation, as an addition reaction.

Preferably CF₃CF₂CH₂Cl is then exposed to reaction conditions effective to produce a reaction product containing one or more of the desired fluorolefins, such as 1234yf. In one preferred aspect of the present invention, the conversion step comprises a reaction that is sometimes referred to herein as a reduction reaction and in other aspects as a dehydrohalogenation reaction. Preferred aspects of each of the preferred steps are described below.

### I. ADDITION REACTION

CF₂=CF₂ (sometimes referred to herein as "TFE") is reacted with CH₂FCl.

In certain preferred embodiments, the addition step comprises contacting CH₂FCl:TFE at a mole ratio of from 1:1 to 200:1, more preferably from 1:1 to 100:1 and even more preferably of from 2:1 to 3:1. In preferred embodiments, the CH₂FCl:TFE mole ratio of the feeds to the reactor are from 1:1 to 200:1, more preferably from 1:1 to 100:1 1 and even more preferably from 1.5:1 to 2:1.

It is contemplated that this reaction step can be carried out in the liquid phase or in the gas phase, or a combination of liquid/gas phases, and it is further contemplated that the reaction can be carried out batch wise, continuous, or a combination of these.

Thus, it is contemplated that the addition step may be performed using a wide variety of process parameters and process conditions in view of the overall teachings contained herein. However, it is preferred in certain embodiments that this reaction step comprise a gas phase reaction, preferably in the presence of catalyst, supported on carbon or unsupported, preferably a metal-based catalyst, such as antimony-based catalysts (such as SbF₃, SbF₅, and partially flourinated SbCl₃ or SbCl₅) aluminum-based catalyst (such as AlCl₃), iron-based catalyst such FeCl₃ including such catalysts on a carbon or other appropriate support. It is expected that many other catalysts may be used depending on the requirements of particular embodiments, and of course, two or more any of these catalysts, or other catalysts not named here, may be used in combination.

The gas phase addition reaction may be conducted, for example, by introducing a gaseous form of TFE and CH₂FCl into a suitable reaction vessel or reactor. Preferably the vessel is comprised of materials which are resistant to corrosion, such as Hastelloy, Inconel, Monel and/or fluoropolymers linings. Preferably the vessel contains catalyst, for example a fixed or fluid catalyst bed, packed with a suitable addition catalyst, with suitable means to heat the reaction mixture to the desired reaction temperature.

While it is contemplated that a wide variety of reaction temperatures and pressures may be used, depending on relevant factors such as the catalyst being used and the most desired reaction product, it is generally preferred that at least a portion of the addition step is carried out at a reaction temperature of from 5°C to 1000°C, more preferably 5°C to 500°C, more preferably 5°C to 200°C and even more preferably from 40°C to 60°C for reactors which are preferably maintained at a pressure of from 7kPa to 10 Mpa gauge (1 to 1500 psig), and even more preferably from 140 to 280 kPa gauge (20 to 40 psig).

In certain preferred embodiments, TFE and CH₂FCl are introduced into an appropriate reaction vessel in the form of a gas and the reactor is preferably maintained at a temperature of 50°C and the reactor is preferably maintained at a pressure of 200 kPa (30 psig).

In preferred embodiments the conversion of TFE is preferably at least 15%, more preferably at least 20%, and selectivity to CF₃CF₂CH₂Cl is preferably at least 50%, more preferably at least 70%, and even more preferably at least 75%.

### II. FORMATION OF 1234YF

The methods of the present invention preferably comprise converting CF₃CF₂CH₂Cl to 1234yf.

In certain preferred embodiments, the present converting step is carried out under conditions effective to provide a CF₃CF₂CH₂Cl conversion of at least 40%, more preferably at least 55%, and even more preferably at least 70%. In certain preferred embodiments the conversion is at least 90%, and more preferably 100%. Further in certain preferred embodiments, the conversion of CF₃CF₂CH₂Cl to produce 1234yf is conducted under conditions effective to provide a 1234yf selectivity of at least 25%, more preferably at least 40%, more preferably at least 70%, and even more preferably at least 90%.

This reaction step can be carried out in the liquid phase or in the gas phase, or in a combination of gas and liquid phases, and it is contemplated that the reaction can be carried out batch wise, continuous, or a combination of these.

### A. GAS PHASE DEHYDROHALOGENATION

One preferred reaction step in accordance may be described by those reactions in which CF₃CF₂CH₂Cl undergoes gas-phase deydrohalogenation.

For example, an embodiment involving CF₃CF₂CH₂Cl may be shown to proceed by the following reaction, where the reducing agent is hydrogen:

**CF₃CF₂CH₂Cl + H₂ → CF₃CF=CH₂ + HCl + HF**

Although applicant does not intend to be bound to or limited by any particular theory of operation, is believed that in some embodiments the above noted reaction proceeds by the formation CF₃CF₂CH₃, which is generated as an intermediate or byproduct of the reaction with hydrogen, methane or other dehydrogenating agent. In accordance with such theory, the intermediate CF₃CF₂CH₃ compound is then converted to HFO-1234yf, under the existing reaction conditions, and preferably on the surface of the catalyst.

In an alternative to the above reaction, the reducing agent comprises methane and the reaction is represented, without limitation, as follows:

**CF₃CF₂CH₂Cl + CH₄ → CF₃CF=CH₂ + CH₃Cl + HF**

In certain preferred embodiments, the stream containing CF₃CF₂CH₂Cl is preheated, primarily to avoid condensation, to a temperature of from 50°C to 90°C, preferably 60°C to 70°C, and introduced into a reaction vessel. The appropriate amount of the reducing agent, which is preferably from 0.1% to 500% of the stoichometric amount, is then added to the reaction vessel. Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable dehydrohalogenation catalyst, with suitable means to heat the reaction mixture to the desired reaction temperature.

Thus, it is contemplated that the dehydrohalogenation reaction step may be preformed using a wide variety of process parameters and process conditions in view of the overall teachings contained herein. However, it is preferred in certain embodiments that this reaction step comprise a gas phase reaction, preferably in the presence of catalyst, and even more preferably a carbon- and/or metal-based catalyst, such as activated carbon, palladium on carbon, palladium-based catalyst (including palladium on carbon and palladium on aluminum oxides), and ruthenium-based catalysts (including ruthenium on aluminum oxides). It is expected that many other catalysts may be used depending on the requirements of particular embodiments in view of the teachings contained herein. Of course, two or more any of these catalysts, or other catalysts not named here, may be used in combination.

In general it is preferred that the catalysts are fluorinated, preferably for a period of from several hours (eg, 6 hours). In preferred embodiments, fluorination of the catalysts comprises exposing the catalyst to a stream of HF at reaction temperature and under slight pressure, for example 30 kPa - 1.0 Mpa absolute (5-150 psia).

The gas phase dehydrohalogenation reaction may be conducted, for example, by introducing a gaseous form of CF₃CF₂CH₂Cl and a gaseous form of the reducing agent (and/or dehydrohalogenation agent), into a suitable reaction vessel or reactor. Preferably the vessel is comprised of materials which are resistant to corrosion as Hastelloy, Inconel, Monel and/or fluoropolymers linings. Preferably the vessel contains catalyst, for example a fixed or fluid catalyst bed, packed with a suitable reduction/dehydrohalogenation catalyst, with suitable means to heat the reaction mixture to the desired reaction temperature.

While it is contemplated that a wide variety of reaction temperatures may be used, depending on relevant factors such as the catalyst being used and the most desired reaction product, it is generally preferred that the reaction temperature for the dehydrohalogentation step is from 400°C to 800°C, preferably 400°C to 700°C. For such embodiments in which the reducing agent comprises, and even more preferably consists essentially of hydrogen, the reaction temperature for the dehydrohalogentation step is preferably from 450°C to 600°C, more preferably from 450°C to 550°C. For such embodiments in which the reducing agent comprises, and even more preferably consists essentially of methane, the reaction temperature for the dehydrohalogentation step is preferably from 500°C to 700°C, more preferably from 600°C to 700°C.

In general it is also contemplated that a wide variety of reaction pressures may be used, depending again on relevant factors such as the specific catalyst being used and the most desired reaction product. The reaction pressure can be, for example, superatmospheric, atmospheric or under vacuum, and in certain preferred embodiments is from 100 kPa to 830 kPa absolute (15 to 120 psia).

In certain embodiments, an inert diluent gas, such as nitrogen, may be used in combination with the other reactor feeds. When such a diluent is used, it is generally preferred that CF₃CF₂CH₂Cl comprises from 5% to greater than 99% by weight based on the combined weight of diluent and CF₃CF₂CH₂Cl.

It is contemplated that the amount of catalyst use will vary depending on the particular parameters present in each embodiment. In certain preferred embodiments, the contact time is from 0.1 seconds to 1000 second, and preferably from 2 seconds to 50 seconds. For embodiments in which the reducing agent comprises or consists essentially of hydrogen, and particularly where the desired product is HFO-1234yf, applicants have found that it is preferred to use as the catalyst a carbon-based catalyst, such as activated carbon, or palladium-based catalyst, or a catalyst comprising palladium and carbon, such as a palladium on carbon catalyst.

For embodiments in which the reducing agent comprises or consists essentially of methane, and particularly where the desired product is HFO-1234yf, applicants have found that it is preferred to use as the catalyst a carbon-based catalyst, such as activated carbon, or a catalyst based on a Period 6 metal (particularly Cs and Ba) such as BaNO₃ or CsNO₃ (including in combination with aluminum-based catalyst or catalyst support, such as Al₂O₃), or Ni-based catalyst (such as Ni mesh) and combinations of these.

Preferably in such dehydrofluorination embodiments as described in this section, the conversion of CF₃CF₂CH₂Cl is at least 50%, more preferably at least 65%, and even more preferably at least 90%. Preferably, the selectivity to HFO-1234yf is at least 70%, more preferably at least 80% and more preferably at least 90%.

### EXAMPLES

Additional features of the present invention are provided in the following examples.

### Examples 1 - 11

These examples illustrate gas phase dehydrohalogenation of CF₃CF₂CH₂Cl (HFC-235cb) to CF₃CF=CH₂ (1234yf). A 56 cm (22-inch) (1.25 cm, 1/2-inch, diameter) Monel tube reactor is charged with 120 cc of catalyst, as specified in Table 1 below. The reactor is mounted inside a heater with three zones (top, middle and bottom). The inlet of the reactor is connected to a pre-heater, which was kept at 300°C by electrical heating. Organic (235cb) is fed from a cylinder kept at 65°C. A flow of reducing agent comprising hydrogen gas and the inert N₂ gas is maintained at a rate as indicated in Table 1 below. The reactor temperature is brought to the temperature indicated in the table. The HFC-235cb is passed through gas-flow controllers into a preheater maintained a temperature of 300°C. The gas stream coming out of the preheater is passed through the catalyst bed at the desired temperature over a specified period of time and at a pressure of from 17 kPa-37kPa gauge (2.5 - 5.3 psig). An on-line GC and a GCMS are used to analyze samples taken at the reactor exit line at regular time intervals. Finally, the reactor effluent is introduced into a 20 - 60 % KOH scrubber solution, and the effluent from the scrubber solution is then condensed to collect the products. The desired product CF₃CF=CH₂ (1234yf) is then isolated from the mixture by distillation. The conversion of HFC-235cb is from 50% to 100% and the selectivity to HFO-1234yf is from 60% to 100%, depending on the reaction conditions. The major byproducts were CF₃CF₂CH₃ (HFC-245cb), CF₃CF₂Cl(CFC-115), CF₃Cl(CFC-13), and CF₃CF=CHCl.

The results are shown in Table 1 below.

**Table 1**

| CF₃CF₂CH₂Cl (HFC-235cb) + H₂ → CF₃CF=CH₂ (1234yf) + HCl + HF | | | | | | | | |
|---|---|---|---|---|---|---|---|---|
| **Example# / Catalyst** | **T, °C** | **H2/N2, Sccm** | **HFC-235cb, sccm** | **% Conv. of 235cb** | **% Selec. to 1234yf** | **% Selec. to 245cb** | **% Selec. to R*** | **% Selec. to R**** |
| Example 1/ A | 450 | 58/0 | 52 | 76 | 60 | 31 | 4 | 4 |
| Example 2/ A | 500 | 61/0 | 54 | 100 | 90 | 9 | 0 | 1 |
| Example 3/ A | 550 | 61/0 | 54 | 100 | 84 | 6 | 1 | 6 |
| Example 4/ A | 600 | 63/0 | 57 | 97 | 63 | 12 | 0 | 24 |
| Example 5/ B | 520 | 41/100 | 96 | 80 | 60 | 20 | 5 | 10 |
| Example 6/ B | 550 | 41/100 | 138 | 58 | 36 | 40 | 7 | 16 |
| Example 7/ C | 500 | 61/0 | 54 | 87 | 69 | 20 | 4 | 6 |
| Example 8/ D | 500 | 61/0 | 54 | 80 | 63 | 26 | 3 | 7 |
| Example 9/ E | 500 | 61/0 | 63 | 100 | 73 | 16 | 2 | 8 |
| Example 10/ F | 500 | 61/0 | 76 | 91 | 30 | 27 | 10 | 30 |
| Example 11/ G | 500 | 61/0 | 100 | 80 | 28 | 29 | 8 | 32 |
| Catalysts (100 cc): A is Calgon activated carbon; B is Shiro-Saga activated carbon; C is Aldrich activated carbon; D is NORIT RFC 3 activated carbon; E is 0.5 wt% Pd/C; | | | | | | | | |
| F is 0.5 wt% Pd/A1203 ; G is 0.5wt% Ru/A1203 | | | | | | | | |
| Byproducts: R* is CF₃CF=CHCl and R** is combination of CF3Cl and CF₃CF₂Cl | | | | | | | | |

### Examples 12 - 15

These examples illustrate gas phase hydrodehydrochlorination of CF₃CF₂CH₂Cl to CF₃CF=CH₂ (HFO-1234yf) using methane as the reducing agent. The protocol of Examples 1 -11 is repeated except as indicated in Table 2 below.

**Table 2**

| CF₃CF₂CH₂Cl (235cb) + CH₄ → CF₃CF=CH₂ (1234yf) + CH₃Cl + HF | | | | | | |
|---|---|---|---|---|---|---|
| **Example#** / **Catalyst** | **T, °C** | **P, psig** | **CH₄, sccm** | **HFC-235cb, sccm** | **% Conv. of 235cb** | **% Selec. to 1234yf** |
| Example 12/ H | 560 | 10 | 50 | 50 | 17 | 0 |
| Example 13/ I | 650 | 8 | 100 | 86 | 46 | 26 |
| Example 14/ J | 650 | 7 | 97 | 83 | 52 | 31 |
| Example 14*/ K | 650 and 500 * | 8 | 96 | 87 | 54 | 40 |
| Example 15/ L | 585 | 3 | 79 | 100 | 77 | 33 |
| Catalysts (100 cc): H is activated carbon; I3wt% | | | | | | |
| BaNO₃/Al₂O₃; J is 3wt% BaNO₃/Al₂0₃ with 0.5wt% CsNO₃ used as a promoter; K is a two zone reaction with two catalysts, specifically 50 cc CsNO₃ promoted with BaNO₃/Al₂O₃ is used at 650°C in the first zone and 50 cc Calgon activated carbon is used at 500°C in the second zone; L is Ni mesh. | | | | | | |
| * two zone reaction | | | | | | |

### Example 16 (Reference)

This example illustrates the liquid phase reduction of CF₃CF=CHCl to CF₃CF=CH₂ (1234yf) with Pd(PPh₃)₄ catalyst. A 250 mL Parr reactor/autoclave is charged with 30 mL tetrahydrofuran to which 0.5 grams (0.04 mmol) [tetrakis(triphenylphosphine)palladium(0), [ Pd(PPh₃)₄], 10.0 grams of ammonium formate (158 mmol), and 11.4 grams cold (0-10 °C) CF3CF=CHCl (77 mmol) are added under nitrogen. The reactor is sealed immediately, cooled to -30 to 40°C, and partially evacuated. The contents in the Parr reactor are brought to room temperature and gradually heated to 100°C with stirring. The reactants are maintained at this temperature for 24 hours. During this time, the pressure in the reactor is increased to approximately 1200-1400 kPa gauge (180-200 psig). The reactor is then cooled to 25°C and the volatile materials are collected in an evacuated metal cylinder.

Gas chromatographic (GC) analysis of the volatile materials indicated CF₃CF=CH₂ as the main product with trace amounts of CF₃CF=CHCl and carbon dioxide. The reaction mixture is cooled to 0°C and filtrate is analyzed by gas chromatograph (GC) which indicates approximately a 40% conversion of CF₃CF=CHCl to CF3CF=CH₂. Identity of CF₃CF=CH₂ is confirmed by comparison with a known sample. Purification of the product is accomplished by passing the product through a cold trap to remove CO₂ and unreacted starting material at -70°C and then distillation.

### Example 17 (Reference)

This example illustrates the liquid phase reduction of CF₃CF=CHCl to CF₃CF=CH₂ (HFO-1234yf) with Pd₂(dba)₃ catalyst. A 250 mL Parr reactor/autoclave was charged with 30 mL tetrahydrofuran (30 mL) to which 0.456 grams of tris(dibenzlideneacetone)dipalladium(0), Pd₂(dba)₃ (2.0 mmol), 0.8 grains of tributyl phosphine (2.0 mmol), 8.0 grams of ammonium formate (126 mmol), and 11.4 grams of cold (5-10 °C) CF₃CF=CHCl (77 mmol) are added under nitrogen. The reactor is sealed immediately, cooled to the range of from -30°C to -40 °C, and partially evacuated. The contents in the Parr reactor are brought to room temperature and then gradually heated to 100°C with stirring. The reactants are maintained at this temperature for 24 hours during which the internal pressure rises to approximately 1,400 kPa gauge (200 psig). The reactor is then cooled to 25°C and the volatile materials are passed through a trap at -70°C to -78°C and collected in a cold evacuated metal cylinder. Gas chromatographic (GC) analysis of the volatile materials collected indicate CF₃CF=CH₂ as the main product with trace amounts of CF₃CF=CHCl and carbon dioxide. The reaction mixture was cooled to 0 °C and filtrate was analyzed by GC which indicated approximately a 35% conversion of CF₃CF=CHCl to CF₃CF=CH₂.

### Example 18 (Reference)

This example illustrates the liquid phase reduction of CF₃CF=CHCl to CF₃CF=CH₂ (HFO-1234yf) with Pd₂(dba)₃ catalyst. Example 17 is repeated except that dioxane is substituted for tetrahydrafuran as the solvent. The result is essentially the same as Example 17.

### Example 19 (Reference)

This example illustrates the liquid phase reduction of CF₃CF=CHCl to CF₃CF=CH2 (HFO-1234yf) using Pd₂(dba)₃·CHCl₃ catalyst. The reaction is carried out as in Example 17 except that an equivalent amount of catalyst Pd₂(dba)₃ is substituted by tris(dibenzlideneacetone)dipalladium (0) chloroform complex, Pd₂(dba)₃•CHCl₃. The extent of conversion of CF₃CF=CHCl to CF₃CF=CH₂ was essentially the same as in Example 17.

### Example 20 (Reference)

This example illustrates the liquid phase reduction of CF₃CF=CHCl to CF₃CF=CH₂ (HFO-1234yf) with Pd(PPh₃)₄ catalyst in tetrahydrofuran. A 250 mL Parr Reactor/autoclave was charged with 30 mL of tetrahydrofuran to which 0.5 grams of tetrakis(triphenylphosphine)palladium(0), Pd(PPh₃)₄(0.04 mmol), 10 grams of ammonium formate (158 mmol), and 11.4 grams of CF₃CF=CHCl (61 mmol) were added under nitrogen. The reactor is sealed immediately, cooled to -30 to -40°C, and partially evacuated. The contents in the Parr reactor were brought to room temperature and gradually heated to 100°C with constant stirring. The reactants are maintained at this temperature for 24 hours. The reactor was then cooled to 25°C and the volatile materials were collected in an evacuated metal cylinder. Gas chromatographic (GC) analysis of the volatile materials indicated that CF₃CF=CH₂ and CF₃CH=CHCI are present at a ratio of 57:42. Carbon dioxide was also present. The reaction mixture was cooled to 0°C and filtered under pressure, the filtrate was analyzed by GC which indicated a 40% conversion of CF₃CF=CHCl to CF₃CH=CH₂.

### Examples 21 - 28 (Reference)

These examples illustrate gas phase dehydrohalogenation of CF₃CF₂CH₃ (HFC-245cb) to CF₃CF=CH₂ (HFO-1234yf). A 22-inch (1/2-inch diameter) Monel tube reactor is charged with 120 cc of catalyst, as specified in Table I below. The reactor is mounted inside a heater with three zones (top, middle and bottom). The inlet of the reactor is connected to a pre-heater, which was kept at 300°C by electrical heating. Organic (HFC-245cb) is fed from a cylinder kept at 65°C. A flow of reducing agent comprising hydrogen gas is maintained at a rate as indicated in Table 3 below. The reactor temperature is brought to the temperature indicated in the table. The HFC-245cb is passed through gas-flow controllers into a preheater maintained a temperature of 300°C. The gas stream coming out of the preheater is passed through the catalyst bed at the desired temperature over a specified period of time and at a pressure of from 17 kPa - 37 kPa gauge (2.5 - 5.3 psig). An on-line GC and a GCMS are used to analyze samples taken at the reactor exit line at regular time intervals. Finally, the reactor effluent is introduced into a 20 ∼ 60 % KOH scrubber solution, and the effluent from the scrubber solution is then condensed to collect the products. The desired product CF₃CF=CH₂ (HFO-1234yf) is then isolated from the mixture by distillation. The conversion of HFC-245cb is from 30% to 70% and the selectivity to HFO-1234yf is from 90% to 100%, depending on the reaction conditions.

The results are shown in Table 3 below.

**Table 3**

| CF₃CF₂CH₃ (HFC-245cb) → CF₃CF=CH₂ (1234yf) | | | | | |
|---|---|---|---|---|---|
| **Example# / Catalyst** | **T, °C** | **H2/N2, sccm** | **HFC-245cb, sccm** | **% Conv. of 245cb** | **% Selec. to 1234yf** |
| Example 21/ M | 575 | 0 | 65 | 79 | 63 |
| Example 22/ N | 575 | 0 | *68* | 82 | 57 |
| Example 23/ O | 575 | 0 | 73 | 73 | 61 |
| Example 24/ P | 575 | 0 | 68 | 84 | 59 |
| Example 25/ P | 575 | 20 | 68 | 89 | 73 |
| Example 26/ Q | 550 | 0 | 69 | 92 | 53 |
| Example 27/ R | 550 | 0 | 67 | 93 | 33 |
| Example 28/ S | 550 | 0 | 69 | 73 | 46 |
| Catalysts (100 cc): M is NORIT RFC 3; N is Shiro-Saga activated carbon; O is Aldrich activated carbon; P is Calgon activated carbon; Q is 0.5 wt% Pd/C; R is 0.5 wt% PVC; S is Ni-mesh | | | | | |

### Example 29

This example illustrates the addition formation of compounds of formula CF₃CF₂CH₂Cl by the reaction of TFE with CH₂FCl in a gas phase reaction. Into a ½ inch flow reactor (Monel) 50 grams of freshly prepared catalyst (as indicated below) are charged. CF₂=CF₂ (TFE) and CH₂FCl (R31) are passed through a mass flow controller with a desired flow rate (as indicated below) to the preheater from respective cylinders connected with regulators. The preheater was connected to the reactor and always kept 10°C below the reactor temperature. The reactor was uniformly heated to the desired temperature by an external heating element with an automatic control. The exit line from the reactor was connected to an on-line GC and GCMS for analysis. A 15 wt% KOH scrubber solution was used at 50°C to neutralize acids coming out from the reactor. The gas stream coming out of the scrubber solution was then condensed in a cylinder under liquid N2 and then finally fractionated (distilled) to isolate products. SbF₅/C and AlCl₃/C are used as the catalyst. At 50°C and under 200 kPa gauge (30 psig) reactor pressure, when 50 sccm of TFE and 150 sccm of R31 were passed over SbF5/C to achieve a 26% conversion of TFE and an 82% selectivity to CF₃CF₂CH₂Cl. When AlC13/C is used as the catalyst, a 35% conversion and 78% selectivity to CF₃CF₂CH₂Cl was obtained.

### Example 30 (Reference)

This example illustrates the reaction of CTFE with CH₂FCl in a gas phase reaction. Example 29 is repeated except that CTFE is used in place of TFE. The major reaction products include CF₃CCIFCH₂Cl and F₂ClCCF₂CH₂Cl at a 21% conversion (CTFE) level.

### Example 31

This example illustrates the formation of compounds of formula CF₃CF₂CH₂Cl by the reaction of TFE with CH₂FCl in a gas phase reaction. Into a 300 ml autoclave, 0.1 mol C₂F₄ was reacted with 0.2 mol CH₂ClF in the presence of 0.05 mol of AlCl₃ at 20-30° for 3 hr to give 60% yield to CF₃CF₂CH₂Cl which was then isolated and purified by distillation.

### Example 32

This example illustrates the formation of compounds of formula CF₃CF₂CH₂Cl by the reaction of TFE with CH₂FCl in a gas phase reaction. Into a 300 ml autoclave, 0.1 mol C₂F₄ was reacted with 0.2 mol CH₂ClF in the presence of 0.05 mol of AICl₃ at 20-30° for 3 hr to give 60% yield to CF₃CF₂CH₂Cl which was then isolated and purified by distillation.

### Example 33 (Reference)

This example illustrates the reaction of CTFE with CH₂FCl in a gas phase reaction. Into a 300 ml autoclave, 0.1 mol CF₂=CFCl was reacted with 0.2 mol CH₂CIF in the presence of 0.05 mol of AlCl₃ at 20-30° for 3 hr to give 60% yield to CF₃CClFCH₂Cl and F₂ClCF₂CH₂Cl which was then isolated and purified by distillation.

## Claims

1. A method of preparing fluorinated organic compounds comprising:
reacting tetrafluoroethylene with CH₂FCl under conditions effective to produce CF₃CF₂CH₂Cl.

2. The method of claim 1, wherein reacting tetrafluoroethylene with CH₂FCl comprises a liquid-phase reaction.

3. The method of claim 1 wherein reacting tetrafluoroethylene with CH₂FCl comprises a gas-phase reaction.

4. The method of claim 3, wherein tetrafluoroethylene is reacted with CH₂FCl in the presence of a catalyst.

5. The method of claim 4 wherein said catalyst is selected from the group consisting of supported or unsupported antimony-based catalysts, supported or unsupported aluminium-based catalysts, and supported or unsupported iron-based catalysts, preferably wherein the supported or unsupported catalyst comprises one or more of SbF₃, SbF₅, partially flourinated SbCl₃, partially flourinated SbCl₅, AlCl₃ and FeCl₃, more preferably wherein the catalyst is supported on carbon.

6. The method of claim 4 or claim 5, wherein the catalyst is present in a fixed or fluid catalyst bed.

7. The method of any one of claims 3 to 6, wherein the reaction temperature is from 5 to 1000°C and the reactor pressure is from 7kPa to 10 Mpa gauge (from 1 to 1500 psig), preferably wherein the reaction temperature is from 5°C to 500°C.

8. The method of any preceding claim wherein the method further comprises converting CF₃CF₂CH₂Cl to CF₃CF=CH₂ in a gas phase reaction.

9. The method of claim 8 wherein at least one of said reacting step or said converting step is a continuous process.

10. The method of claim 8, wherein converting CF₃CF₂CH₂Cl to CF₃CF=CH₂ is conducted in the presence of from 0.1% to 500% of reducing agent, based upon the number of moles of CF₃CF₂CH₂Cl, preferably wherein the reaction temperature for converting CF₃CF₂CH₂Cl to CF₃CF=CH₂ is from 400 to 800°C, more preferably wherein the reaction temperature for converting CF₃CF₂CH₂Cl to CF₃CF=CH₂ is from 400 to 700°C.

11. The method of claim 10, wherein the reducing agent comprises at least one of hydrogen and methane.

12. The method of claim 11, wherein the reducing agent comprises hydrogen and the reaction temperature for converting CF₃CF₂CH₂Cl to CF₃CF=CH₂ is from 450 to 600°C.

13. The method of claim 11, wherein the reducing agent comprises methane and the reaction temperature for converting CF₃CF₂CH₂Cl to CF₃CF=CH₂ is from 500 to 700°C.

14. The method of any one of claims 8 to claim 13, wherein converting CF₃CF₂CH₂Cl to CF₃CF=CH₂ is carried out in the presence of a catalyst.

15. The method of claim 14, wherein the catalyst is selected from carbon- and/or metal based catalysts, palladium-based catalysts, ruthenium-based catalysts and combinations thereof, preferably wherein the catalyst is selected from activated carbon, palladium on carbon, palladium on aluminium oxides, ruthenium on aluminium oxides and combinations thereof.

## Patentansprüche

1. Verfahren zur Herstellung von fluorierten organischen Verbindungen, umfassend:
Umsetzung von Tetrafluorethylen mit CH₂FCl unter Bedingungen, die zur Herstellung von CF₃CF₂CH₂Cl wirksam sind.

2. Verfahren nach Anspruch 1, wobei die Umsetzung von Tetrafluorethylen mit CH₂FCl eine Flüssigphasenreaktion umfasst.

3. Verfahren nach Anspruch 1, wobei die Umsetzung von Tetrafluorethylen mit CH₂FCl eine Gasphasenreaktion umfasst.

4. Verfahren nach Anspruch 3, wobei Tetrafluorethylen mit CH₂FCl in Gegenwart eines Katalysators umgesetzt wird.

5. Verfahren nach Anspruch 4, wobei der Katalysator ausgewählt ist aus der Gruppe, bestehend aus geträgerten oder nicht geträgerten Katalysatoren auf Antimonbasis, aus geträgerten oder nicht geträgerten Katalysatoren auf Aluminiumbasis und aus geträgerten oder nicht geträgerten Katalysatoren auf Eisenbasis, wobei vorzugsweise der geträgerte oder nicht geträgerte Katalysator einen oder mehrere von SbF₃, SbF₅, teilweise fluoriertem SbCl₃, teilweise fluoriertem SbCl₅, AlCl₃ und FeCl₃ umfasst, wobei der Katalysator vorzugsweise auf Kohlenstoff geträgert ist.

6. Verfahren nach Anspruch 4 oder Anspruch 5, wobei der Katalysator in einem festen oder flüssigen Katalysatorbett vorliegt.

7. Verfahren nach einem der Ansprüche 3 bis 6, wobei die Reaktionstemperatur von 5 bis 1.000 °C und der Reaktordruck von 7 kPa bis 10 MPa (von 1 bis 1.500 psig) beträgt, wobei vorzugsweise die Reaktionstemperatur von 5 °C bis 500 °C beträgt.

8. Verfahren für jeden vorhergehenden Anspruch, wobei das Verfahren ferner die Umwandlung von CF₃CF₂CH₂Cl in CF₃CF=CH₂ in einer Gasphasenreaktion umfasst.

9. Verfahren nach Anspruch 8, wobei mindestens einer der Reaktionsschritte oder der Umwandlungsschritt ein kontinuierlicher Prozess ist.

10. Verfahren nach Anspruch 8, wobei die Umwandlung von CF₃CF₂CH₂Cl zu CF₃CF=CH₂ in Gegenwart von 0,1 % bis 500 % Reduktionsmittel, bezogen auf die Molzahl von CF₃CF₂CH₂Cl, durchgeführt wird, wobei vorzugsweise die Reaktionstemperatur zur Umwandlung von CF₃CF₂CH₂Cl zu CF₃CF=CH₂ von 400 bis 800 °C beträgt, wobei noch bevorzugter die Reaktionstemperatur zur Umwandlung von CF₃CF₂CH₂Cl in CF₃CF=CH₂ von 400 bis 700 °C beträgt.

11. Verfahren nach Anspruch 10, wobei das Reduktionsmittel mindestens eines von Wasserstoff und Methan umfasst.

12. Verfahren nach Anspruch 11, wobei das Reduktionsmittel Wasserstoff umfasst und die Reaktionstemperatur zur Umwandlung von CF₃CF₂CH₂Cl in CF₃CF=CH₂ von 450 bis 600 °C beträgt.

13. Verfahren nach Anspruch 11, wobei das Reduktionsmittel Methan umfasst und die Reaktionstemperatur zur Umwandlung von CF₃CF₂CH₂Cl in CF₃CF=CH₂ von 500 bis 700 °C beträgt.

14. Verfahren nach einem der Ansprüche 8 bis 13, wobei die Umwandlung von CF₃CF₂CH₂Cl in CF₃CF=CH₂ in Gegenwart eines Katalysators durchgeführt wird.

15. Verfahren nach Anspruch 14, wobei der Katalysator ausgewählt ist aus Katalysatoren auf Kohlenstoff- und/oder Metallbasis, Katalysatoren auf Palladiumbasis, Katalysatoren auf Rutheniumbasis und Kombinationen davon, wobei vorzugsweise der Katalysator ausgewählt ist aus Aktivkohle, Palladium auf Kohlenstoff, Palladium auf Aluminiumoxiden, Ruthenium auf Aluminiumoxiden und Kombinationen davon.

## Revendications

1. Procédé de préparation de composés organiques fluorés comprenant :
la réaction de tétrafluoroéthylène avec du CH₂FCl dans des conditions efficaces pour produire du CF₃CF₂CH₂Cl.

2. Procédé de la revendication 1, dans lequel la réaction de tétrafluoroéthylène avec du CH₂FCl comprend une réaction en phase liquide.

3. Procédé de la revendication 1 dans lequel la réaction de tétrafluoroéthylène avec du CH₂FCl comprend une réaction en phase gazeuse.

4. Procédé de la revendication 3, dans lequel on fait réagir du tétrafluoroéthylène avec du CH₂FCl en présence d'un catalyseur.

5. Procédé de la revendication 4 dans lequel ledit catalyseur est choisi dans le groupe constitué par les catalyseurs à base d'antimoine sur support ou sans support, les catalyseurs à base d'aluminium sur support ou sans support, et les catalyseurs à base de fer sur support ou sans support, de préférence dans lequel le catalyseur sur support ou sans support comprend un ou plusieurs composés parmi SbF₃, SbF₅, SbCl₃ partiellement fluoré, SbCl₅ partiellement fluoré, AlCl₃ et FeCl₃, mieux encore dans lequel le catalyseur est sur support carboné.

6. Procédé de la revendication 4 ou la revendication 5, dans lequel le catalyseur est présent dans un lit de catalyseur fixe ou fluide.

7. Procédé de l'une quelconque des revendications 3 à 6, dans lequel la température de réaction est de 5 à 1000 °C et la pression dans le réacteur est de 7 kPa à 10 MPa au manomètre (de 1 à 1500 psig), de préférence dans lequel la température de réaction est de 5 °C à 500 °C.

8. Procédé d'une quelconque revendication précédente, le procédé comprenant en outre la transformation de CF₃CF₂CH₂Cl en CF₃CF=CH₂ dans une réaction en phase gazeuse.

9. Procédé de la revendication 8 dans lequel au moins une étape parmi ladite étape de réaction et ladite étape de transformation est un procédé continu.

10. Procédé de la revendication 8, dans lequel la transformation de CF₃CF₂CH₂Cl en CF₃CF=CH₂ est conduite en présence de 0,1 % à 500 % de réducteur, rapporté au nombre de moles de CF₃CF₂CH₂Cl, de préférence dans lequel la température de réaction pour transformer CF₃CF₂CH₂Cl en CF₃CF=CH₂ est de 400 à 800 °C, mieux encore dans lequel la température de réaction pour transformer CF₃CF₂CH₂Cl en CF₃CF=CH₂ est de 400 à 700 °C.

11. Procédé de la revendication 10, dans lequel le réducteur comprend de l'hydrogène et/ou du méthane.

12. Procédé de la revendication 11, dans lequel le réducteur comprend de l'hydrogène et la température de réaction pour transformer CF₃CF₂CH₂Cl en CF₃CF=CH₂ est de 450 à 600 °C.

13. Procédé de la revendication 11, dans lequel le réducteur comprend du méthane et la température de réaction pour transformer CF₃CF₂CH₂Cl en CF₃CF=CH₂ est de 500 à 700 °C.

14. Procédé de l'une quelconque des revendications 8 à 13, dans lequel la transformation de CF₃CF₂CH₂Cl en CF₃CF=CH₂ est réalisée en présence d'un catalyseur.

15. Procédé de la revendication 14, dans lequel le catalyseur est choisi parmi les catalyseurs à base de carbone et/ou de métal, les catalyseurs à base de palladium, les catalyseurs à base de ruthénium et les combinaisons de ceux-ci, de préférence dans lequel le catalyseur est choisi parmi le charbon actif, le palladium sur charbon, le palladium sur oxydes d'aluminium, le ruthénium sur oxydes d'aluminium et les combinaisons de ceux-ci.
